# EUROPEAN PATENT SPECIFICATION

(11) **EP 3 562 395 B1**
(45) Date of publication and mention of the grant of the patent: **04.11.2026**
(21) Application number: 16925387.9
(22) Date of filing: 30.12.2016
(51) Int. Cl.: A61B 5/145, A61B 5/00, A61B 5/11, A61B 5/0205, A61M 5/14, A61M 5/142, A61M 5/172

(54) **SYSTEM FOR CLOSED LOOP CONTROL IN ARTIFICIAL PANCREAS**
SYSTEM FÜR EINEN GESCHLOSSENEN REGELKREIS IN EINER KÜNSTLICHEN BAUCHSPEICHELDRÜSE
SYSTÈME DE COMMANDE EN BOUCLE FERMÉE DANS UN PANCRÉAS ARTIFICIEL

(43) Date of publication of application: 06.11.2019
(73) Proprietor: Medtrum Technologies Inc., Shanghai 201203 (CN)
(72) Inventor: YANG, Cuijun, Shanghai 201203 (CN)
(74) Representative: Becker Kurig & Partner Patentanwälte mbB
(86) International application number: PCT/CN2016/113690
(87) International publication number: WO 2018/120104

(56) References cited:
- CN-A- 102 576 375
- CN-A- 103 889 317
- CN-A- 106 137 214
- JP-A- 2008 253 560
- US-A1- 2007 255 122
- US-A1- 2011 184 380
- US-A1- 2012 078 067
- US-A1- 2015 018 633
- US-A1- 2016 030 669

## Description

### TECHNICAL FIELD

This invention generally relates to medical appliance, and more specifically to a system and method for adjustment of blood glucose related algorithm in a close loop artificial pancreas.

### BACKGROUND

For a normal healthy person, the pancreas produces and releases insulin into the blood stream in response to elevated blood glucose levels. β-cells, which reside in the pancreas, produce and secrete the insulin into the blood stream, as it is needed. If β-cells become incapacitated or die, a condition known as Type I diabetes mellitus, or in some cases if β-cells produce insufficient quantities of insulin, Type II diabetes, then insulin must be provided to the body of the patient from another source.

Traditionally, since insulin cannot be taken orally, insulin has been injected with a syringe. More recently, use of infusion pump therapy has been increasing, especially for delivering insulin for diabetics. For example, external infusion pumps are worn on a belt, in a pocket, or patched to the body of the patient directly, and deliver insulin into the body via an infusion tube with a percutaneous needle or a cannula placed in the subcutaneous tissue. The medicine that is delivered by the infusion pump device can depend on the condition of the patient and the desired treatment plan. However, current insulin pumps and other diabetes treatment instruments are limited in switching between different treatment plans based on different conditions of the patient.

Desired treatment plans using a closed loop algorithm depend on accurate determination of the physical conditions of the patient, especially for the continuously glucose monitoring in tissue fluid whose concentration is easily influenced by the actions of the patient. If a patient is in sleep, due to less activity taking place in the muscle and organs than the normal state, whether she or he needs a low suspend or predictive low suspend requires to be recalculated by adjusted algorithms. Furthermore, some low-priority alerts should be muted to prevent disturbing the patient from sleep. Similarly, if a patient is doing physical exercise, her or his interstitial glucose value sensed by the glucose sensor may fluctuate sharply because of the frequent changes in the tissue fluid, but her or his glucose level should not be determined as abnormal. To realize the closed loop algorithm in an artificial pancreas, the combination of sensing the activity level of the patient and adjusting related algorithms is crucial.

US 2015/018633 A1 discloses a system capable of utilizing data from different monitoring techniques and capable of providing assistance to patients with diabetes at several scalable levels, ranging from advice about long-term trends and prognosis to real-time automated closed-loop control (artificial pancreas). These scalable monitoring and treatment strategies are delivered by a unified system called the Diabetes Assistant (DiAs) platform. The system provides a foundation for implementation of various monitoring, advisory, and automated diabetes treatment algorithms or methods.

US 2012/078067 A1 discloses a structure, method, and computer program product for a diabetes control system with: open-loop or closed-loop control of diabetes that adapts to individual physiologic characteristics and to the behavioral profile of each person.

US 2016/030669 A1 discloses a portable infusion pump communicating with continuous glucose monitor (CGM) to receive continuous feedback relating to a user's blood glucose level during insulin or other medicament therapy and can automatically deliver insulin to a user when the CGM data indicates a need for additional insulin.

US 2011/184380 A1 discloses a system and method for providing closed loop infusion formulation delivery which accurately calculates a delivery amount based on a sensed biological state by adjusting an algorithm's programmable control parameters.

### SUMMARY OF THE INVENTION

The present invention is provided by appended claim 1. The following disclosure serves a better understanding of the present invention. According to the disclosure, to overcome the deficiencies of the prior art, one purpose of the present invention is to provide **a** closed loop artificial pancreas, comprising,a patch pump and a continuous glucose monitoring (CGM) system; a motion sensor set in the CGM system, configured to sense activity levels of a patient and provide corresponding signals; a processor set in the patch pump; and a processor set in the CGM system, configured to, determine physical states of the patient, adjust a plurality of algorithms and send corresponding instructions based partly on the signals to the processor set in the patch pump when the patient is determined in a state of sleep or physical exercise; a processor set in a handset, wherein the handset is adapted to be carried by the patient, wherein the processor set in the handset is configured to adjust related algorithms depending partly on the signals provided by the motion sensor set in the CGM system and send corresponding instructions based partly on the signals to the processor set in the patch pump, wherein a method to realize a closed loop control in the artificial pancreas comprises:sensing the activity level of the patient by the motion sensor set in the CGM system; providing the signals indicative of the activity level of the patient under specific attitudes by the motion sensor set in the CGM system to the processor set in the CGM system, wherein the specific attitudes include standing, sitting, lying, or changing from one of the specific attitudes to another;determining the physical state of the patient according to the activity level via the processor set in the patch pump; adjusting a plurality of algorithms via the processor set in the CGM system depending partly on the signals from the motion sensor set in the CGM system; sending corresponding instructions based on the adjusted algorithms to the processor set in the patch pump from the processor set in the CGM system for automatic operations of the artificial pancreas; and automatically switching the artificial pancreas between different operation modes based at least partly on the adjusted algorithms, wherein the plurality of algorithms includes a low-suspend algorithm, a predicted low-suspend algorithm, and an alert threshold algorithm.

Alternatively, the patch pump and the CGM system are separated from each other; at least one motion sensor is set in the patch pump or the CGM system, and two processers are set in the patch pump and the CGM system respectively.

Alternatively, the patch pump and the CGM system are integrated in a single-needle-integrated artificial pancreas in which both a motion sensor and a processer are set.

The present invention has advantages in the following ways: Firstly, applying the motion sensors in the close loop artificial pancreas enables a comprehensive grasp of the patient's activity levels for a more rational treatment by distinguishing sleep and physical exercise states from the normal state; secondly, adjusting blood glucose related algorithms according to different activity levels and exercise intensities of the patient provides more applicable data and enables automatic operations of the artificial pancreas, such as suspending the delivery under low glucose condition, switching between different operation modes and adjusting insulin delivery of specific operation mode, which reflect the advantages of the closed loop control of artificial pancreas in an all-round way; thirdly, muting some low-priority alerts when sensing the patient in the state of sleep or physical exercise can reduce unnecessary disturbance to the patient making the system more pleasant to use. To sum up, the application of motion sensors in the close loop artificial pancreas enables algorithm adjustments based on different physical states and exercise intensities of the patient to provide more accurate and reliable blood glucose related data that is the basis of desirable treatment plans, and a closed loop artificial pancreas using this method satisfies the requirements of the patient on safety and intelligence of a diabetes treatment system in a more sophisticated way.

### BRIEF DESCRIPTION OF THE DRAWINGS

FIG. 1-3 are schematic diagrams of a patient wearing an artificial pancreas in the present invention
FIG. 4-9 are schematic diagrams of representative method in the embodiments of the present invention

### DETAILED DESCRIPTION

To make the above-mentioned objects, features and advantages of the present invention more obvious and understandable, the embodiments of the present invention are described in the following through specific embodiments.

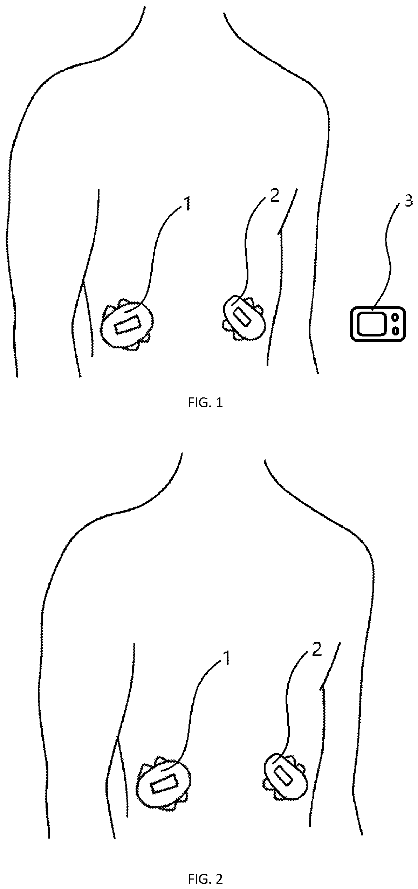

Referring to FIG. 1, an embodiment of the system using the closed loop control in the present invention is provided. FIG. 1 illustrates a patient wearing a CGM system 1 configured to monitor the blood glucose changes in real time and a patch pump 2 configured to deliver insulin to the patient, both equipped with a processor, which communicates with each other via a processor set in a portable handset 3 configured to receive signals, process and display data, and send instructions. At least one motion sensor is set in any one, or two, or all three of the CGM system 1, the patch pump 2 and the handset 3, which will be described in following paragraphs.

Referring to FIG. 2, an embodiment of the system using the closed loop control in the present invention is provided. FIG. 2 illustrates a patient wearing an artificial pancreas comprising two separate components: a CGM system 1 and a patch pump 2, which communicate with each other via their respective processers. At least one motion sensor is set in the CGM system 1 and/or the patch pump 2, which will be described in following paragraphs.

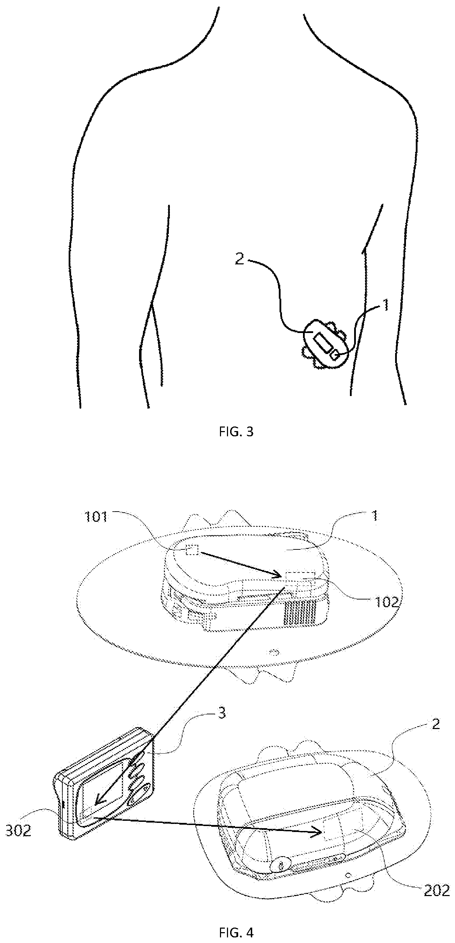

Referring to FIG. 3, an embodiment of the system using the closed loop control in the present invention is provided. FIG. 3 illustrates a patient wearing a single-needle-integrated artificial pancreas integrated by a patch pump 2 and a built-in CGM system 1. A processer is set in the single-needle-integrated artificial pancreas to perform all the functions including receiving signals, processing data, and sending instructions. A motion sensor is also set in the single-needle-integrated artificial pancreas.

Referring to FIG.1 and FIG.4, an embodiment of the present invention is provided. As shown in FIG.4, a motion sensor 101 is set in the CGM system 1, configured to sense activity levels of the patient and send corresponding signals. A processer 102 in the CGM system 1 receives signals from the motion sensor 101 and adjusts related algorithms depending partly on the signals, and the data processed using the adjusted algorithms is sent to a processer 302 in a handset 3. Through further processing, the processer 302 determines whether an operation of the patch pump 2 is needed. If so, the processer 302 sends an instruction to the processer 202 of the patch pump 2 to perform the corresponding operation automatically.

In this embodiment, the motion sensor 101 is a three-axis accelerometer 101.

When the patient is in physical exercise, the start and end of the exercise, as well as the intensity of the exercise can be determined by the equation: ${ACC}_{power} = \sqrt{{{ACC}_{X}}^{2} + {{ACC}_{Y}}^{2} + {{ACC}_{Z}}^{2}}$ Where,
ACC_{power} is the acceleration amplitude of all three axes;
ACC_{X} is the acceleration data of the X axis;
ACCy is the acceleration data of the Y axis;
ACC_{Z} is the acceleration data of the Z axis.

The attitudes of the patient, whether she or he is standing, sitting, lying, or changing from one of these attitudes to another, can be sensed by the three-axis accelerometer 101. In other words, the attitude changes of the patient can be tracked by the three-axis accelerometer 101 in real time. When the patient goes to sleep, the state can be determined by the equation: ${ACC}_{var} = {\left({ACC}_{X}-{ACC}_{X \left|PRE\right.}\right)}^{2} + {\left({ACC}_{Y}-{ACC}_{Y \left|PRE\right.}\right)}^{2} + {\left({ACC}_{Z}-{ACC}_{Z \left|PRE\right.}\right)}^{2}$ Where,
ACCᵥₐᵣ is the acceleration variation of all three axes;
ACC_{X} is the acceleration data of the X axis;
ACCy is the acceleration data of the Y axis;
ACC_{Z} is the acceleration data of the Z axis;
ACC_{X|PRE} is the acceleration data of the X axis at a previous time;
ACC_{Y|PRE} is the acceleration data of the Y axis at a previous time;
ACC_{Z|PRE} is the acceleration data of the Z axis at a previous time.

The algorithms adjusted by the processer 102 comprise but not limited to a low-suspend algorithm, a predictive low-suspend algorithm; and an alert threshold algorithm. As shown in FIG. 4, data processed according to the adjusted algorithms is sent to the handset 3, and the processer 302 in the handset 3 determines whether an operation should be performed automatically by the patch pump 2. If so, the processer 302 sends an instruction to the processer 202 of the patch pump 2. The operations comprise but not limited to a delivery suspend according to the low-suspend algorithm or the predictive low-suspend algorithm, a change of operation modes, an adjustment of the insulin delivery amount of a basal rate mode, and switching the CGM system 1 and the patch pump 2 into an audio-off mode for low priority alerts according to the adjusted algorithm when the patient is determined in a state of sleep or physical exercise.

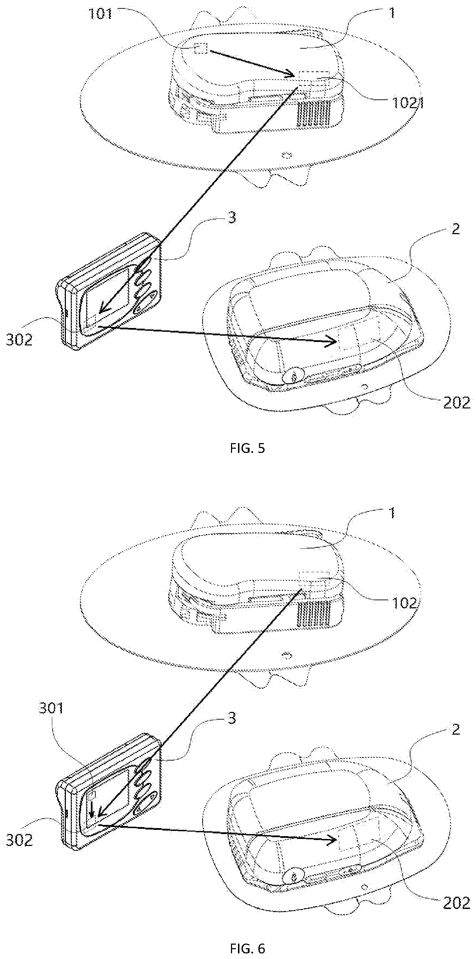

Referring to FIG.1 and FIG.5, an embodiment of the present invention is provided. As shown in FIG.5, a motion sensor 101 is set in the CGM system 1, and the signals provided by the motion sensor 101 are sent to and processed by a processer 302 set in the handset 3 via a transmitting module 1021 set in the CGM system 1. The processer 302 in the handset 3 adjusts related algorithms depending partly on the signals, and determines whether an operation of the patch pump 2 is needed. If so, the processer 302 sends an instruction to the processer 202 of the patch pump 2 to perform the corresponding operation automatically.

Referring to FIG.1 and FIG.6, an embodiment of the present invention is provided. As shown in FIG.6, a motion sensor 301 is set in a portable handset 3 to sense the activity levels of the patient when the patient is carrying the handset 3, and the signals provided by the motion sensor 301 are sent to and processed by a processer 302 set in the handset 3. The processer 302 in the handset 3 adjusts related algorithms depending partly on the signals, and processes the glucose data provided by a processer 102 of the CGM system 1 accordingly. The processer 302 in the handset 3 further determines whether an operation of the patch pump 2 is needed. If so, the processer 302 of the handset 3 sends an instruction to the processer 202 of the patch pump 2 to perform the corresponding operation automatically.

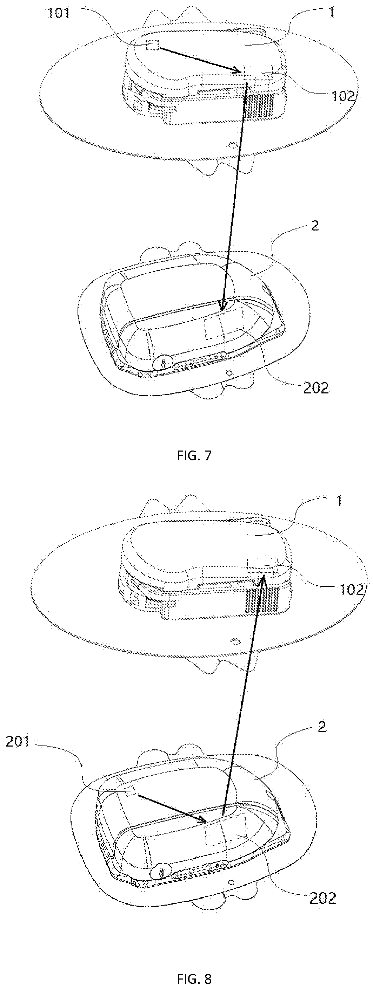

Referring to FIG.2 and FIG.7, an embodiment of the present invention is provided. As shown in FIG.7, a motion sensor 101 is set in the CGM system 1, and the signals provided by the motion sensor 101 are sent to and processed by a processer 102 which is also set in the CGM system 1. The processer 102 adjusts related algorithms depending partly on the signals, and the data processed using the adjusted algorithm is sent to a processer 202 in a patch pump 2 directly. Through further processing, the processer 202 determines whether an operation of the patch pump 2 is needed. If so, an instruction is generated from the processer 202 for the patch pump 2 to perform the corresponding operation automatically.

Referring to FIG.2 and FIG.8, an embodiment of the present invention is provided. As shown in FIG.8, a motion sensor 201 is set in the patch pump 2, and the signals provided by the motion sensor 201 are sent to the processer 202 which is also set in the patch pump 2. The processer 202 determines the physical state of the patient, in specific, whether the patient is in sleep or doing physical exercise, then switches the patch pump 2 to an appropriate operation mode according to the state of the patient automatically. At the same time, the processer 202 sends a signal comprising the physical state and exercise intensity of the patient to the processer 102 of the CGM system to adjust related algorithms, calculating the blood glucose values based on the adjusted algorithms which reflect the actual state of the patient.

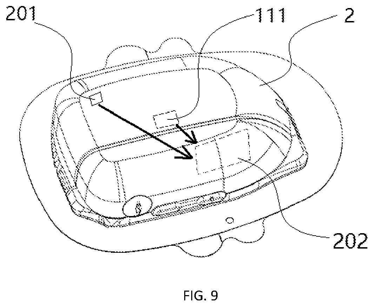

Referring to FIG.3 and FIG.9, an embodiment of the present invention is provided. As shown in FIG.9, a single-needle-integrated artificial pancreas comprises a patch pump 2 and a built-in glucose sensor 111 in FIG. 9. A motion sensor 201 is set in the single-needle-integrated artificial pancreas, and a processer 202 configured to perform all the functions of receiving signals, processing data, and sending instructions is also set in the single-needle-integrated artificial pancreas. The motion sensor 201 senses activity levels of the patient and send corresponding signals to the processer 202. The processer 202 receives signals from the motion sensor 201 and adjusts related algorithms depending partly on the signals to process the glucose data received from the built-in glucose sensor 111, and determines whether an operation of the single-needle-integrated artificial pancreas is needed. If so, an instruction is generated from the processer 202 for the patch pump 2 to perform the corresponding operation automatically, realizing the closed loop control in the single-needle-integrated artificial pancreas.

The above descriptions of the detailed embodiments are only to illustrate the principle and the effect of the present invention which is defined by the scope of the appended claims.

## Claims

1. A closed loop artificial pancreas, comprising,
a patch pump (2) and a continuous glucose monitoring (CGM) system (1);
a motion sensor (101) set in the CGM system (1), configured to sense activity levels of a patient and provide corresponding signals;
a processer (202) set in the patch pump (2); and
a processer (102) set in the CGM system (1), configured to, determine physical states of the patient, adjust a plurality of algorithms and send corresponding instructions based partly on the signals to the processer (202) set in the patch pump (2) when the patient is determined in a state of sleep or physical exercise;
a processer (302) set in a handset (3), wherein the handset (3) is adapted to be carried by the patient, wherein the processer (302) set in the handset (3) is configured to adjust related algorithms depending partly on the signals provided by the motion sensor (101) set in the CGM system (1) and send corresponding instructions based partly on the signals to the processer (202) set in the patch pump (2),
wherein the method used to realize the closed loop control in the artificial pancreas comprises:
sensing the activity level of the patient by the motion sensor (101) set in the CGM system (1);
providing the signals indicative of the activity level of the patient under specific attitudes by the motion sensor (101) set in the CGM system (1) to the processer (102) set in the CGM system (1), wherein the specific attitudes include standing, sitting, lying, or changing from one of the specific attitudes to another;
determining the physical state of the patient according to the activity level via the processer (102) set in the CGM system (1);
adjusting a plurality of algorithms via the processer (102) set in the CGM system (1) depending partly on the signals from the motion sensor (101) set in the CGM system (1);
sending corresponding instructions based on the adjusted algorithms to the processer (202) set in the patch pump (2) from the processer (102) set in the CGM system (1) for automatic operations of the artificial pancreas; and
automatically switching the artificial pancreas between different operation modes based at least partly on the adjusted algorithms,
wherein the plurality of algorithms includes a low-suspend algorithm, a predicted low-suspend algorithm, and an alert threshold algorithm.

2. The closed loop artificial pancreas according to Claim 1, wherein,
the patch pump (2) and the CGM system (1) are separated from each other.

3. The closed loop artificial pancreas according to Claim 1, wherein,
the patch pump (2) and the CGM system (1) are integrated in a single-needle-integrated artificial pancreas;
the motion sensor (101) and the processers (102, 202) are set in the single-needle-integrated artificial pancreas.

4. The closed loop artificial pancreas according to Claim 1, wherein,
the motion sensor (101) comprises one or more from an accelerometer, a gyroscope and an attitude sensor.

5. The closed loop artificial pancreas according to Claim 1, the algorithms are adjusted according to different exercise intensities when the patient is in a physical exercise state.

6. The closed loop artificial pancreas according to Claim 1, wherein
the closed loop artificial pancreas automatically switches to an audio-off mode for low-priority alerts when the patient is determined in the state of sleep or physical exercise.

## Patentansprüche

1. Künstliche Bauchspeicheldrüse mit geschlossenem Regelkreis, umfassend
eine Patch-Pumpe (2) und ein System zur kontinuierlichen Glukoseüberwachung (CGM) (1);
einen in dem CGM-System (1) angeordneten Bewegungssensor (101), der so konfiguriert ist, dass er Aktivitätsniveaus eines Patienten erfasst und entsprechende Signale bereitstellt;
einen in der Patch-Pumpe (2) angeordneten Prozessor (202); und
einen in dem CGM-System (1) angeordneten Prozessor (102), der so konfiguriert ist, dass er körperliche Zustände des Patienten feststellt, eine Vielzahl von Algorithmen anpasst und, teilweise auf der Grundlage der Signale, entsprechende Anweisungen an den in der Patch-Pumpe (2) angeordneten Prozessor (202) sendet, wenn festgestellt wird, dass sich der Patient in einem Zustand von Schlaf oder körperlicher Betätigung befindet;
ein in einem Handgerät (3) angeordneter Prozessor (302), wobei das Handgerät (3) so angepasst ist, dass es von dem Patienten getragen werden kann, wobei der Prozessor (302), der in dem Handgerät (3) angeordnet ist, so konfiguriert ist, dass er zugehörige Algorithmen teilweise in Abhängigkeit von den Signalen, die von dem in dem CGM-System (1) angeordneten Bewegungssensor (101) bereitgestellt werden, anpasst und entsprechende Anweisungen, die teilweise auf den Signalen basieren, an den in der Patch-Pumpe (2) angeordneten Prozessor (202) sendet,
wobei das Verfahren zur Realisierung der Regelung mit geschlossenem Regelkreis in der künstlichen Bauchspeicheldrüse umfasst:
Erfassen des Aktivitätsniveaus des Patienten durch den in dem CGM-System (1) angeordneten Bewegungssensor (101);
Bereitstellen der Signale, die das Aktivitätsniveau des Patienten in bestimmten Körperhaltungen anzeigen, durch den in dem CGM-System (1) angeordneten Bewegungssensor (101) an den in dem CGM-System (1) angeordneten Prozessor (102), wobei die bestimmten Körperhaltungen Stehen, Sitzen, Liegen oder den Wechsel von einer der bestimmten Körperhaltungen in eine andere umfassen;
Bestimmen des körperlichen Zustands des Patienten gemäß dem Aktivitätsniveau mittels des in dem CGM-System (1) angeordneten Prozessors (102);
Anpassen einer Vielzahl von Algorithmen mittels des in dem CGM-System (1) angeordneten Prozessors (102) teilweise in Abhängigkeit von den Signalen des in dem CGM-System (1) angeordneten Bewegungssensors (101);
Senden entsprechender Anweisungen auf der Grundlage der angepassten Algorithmen von dem in dem CGM-System (1) angeordneten Prozessor (102) an den in der Patch-Pumpe (2) angeordneten Prozessor (202) für automatische Vorgänge der künstlichen Bauchspeicheldrüse; und
automatisches Umschalten der künstlichen Bauchspeicheldrüse zwischen verschiedenen Betriebsmodi, zumindest teilweise auf der Grundlage der angepassten Algorithmen,
wobei die Vielzahl von Algorithmen einen Low-Suspend-Algorithmus, einen Predicted-Low-Suspend-Algorithmus und einen Alert-Threshold-Algorithmus umfasst.

2. Künstliche Bauchspeicheldrüse mit geschlossenem Regelkreis nach Anspruch 1, wobei die Patch-Pumpe (2) und das CGM-System (1) voneinander getrennt sind.

3. Künstliche Bauchspeicheldrüse mit geschlossenem Regelkreis nach Anspruch 1, wobei
die Patch-Pumpe (2) und das CGM-System (1) in eine künstliche Bauchspeicheldrüse mit integrierter Einzelnadel integriert sind;
der Bewegungssensor (101) und die Prozessoren (102, 202) in der künstlichen Bauchspeicheldrüse mit integrierter Einzelnadel angeordnet sind.

4. Künstliche Bauchspeicheldrüse mit geschlossenem Regelkreis nach Anspruch 1, wobei
der Bewegungssensor (101) einen oder mehrere aus einem Beschleunigungsmesser, einem Gyroskop und einem Lagesensor umfasst.

5. Künstliche Bauchspeicheldrüse mit geschlossenem Regelkreis nach Anspruch 1, wobei die Algorithmen gemäß unterschiedlichen Betätigungsintensitäten angepasst werden, wenn sich der Patient in einem Zustand körperlicher Betätigung befindet.

6. Künstliche Bauchspeicheldrüse mit geschlossenem Regelkreis nach Anspruch 1, wobei
die künstliche Bauchspeicheldrüse mit geschlossenem Regelkreis automatisch in einen Ton-Aus-Modus für Warnmeldungen mit niedriger Priorität wechselt, wenn festgestellt wird, dass sich der Patient in dem Zustand von Schlaf oder körperlicher Betätigung befindet.

## Revendications

1. Pancréas artificiel en boucle fermée, comprenant :
une pompe patch (2) et un système de surveillance continue de la glycémie (CGM) (1) ;
un capteur de mouvement (101) intégré au système CGM (1), configuré pour détecter les niveaux d'activité d'un patient et fournir des signaux correspondants ;
un processeur (202) intégré à la pompe sous forme de patch (2) ; et
un processeur (102) intégré au système CGM (1), configuré pour déterminer les états physiques du patient, ajuster une pluralité d'algorithmes et envoyer des instructions correspondantes, basées en partie sur les signaux, au processeur (202) intégré à la pompe sous forme de patch (2) lorsque le patient est identifié comme étant en état de sommeil ou d'exercice physique ;
un processeur (302) intégré à un combiné (3), le combiné (3) étant conçu pour être porté par le patient, le processeur (302) intégré au combiné (3) est configuré pour ajuster les algorithmes associés en fonction, entre autres, des signaux fournis par le capteur de mouvement (101) intégré au système CGM (1) et pour envoyer des instructions correspondantes, basées en partie sur ces signaux, au processeur (202) intégré à la pompe patch (2),
dans lequel un procédé permettant de mettre en œuvre une régulation en boucle fermée dans le pancréas artificiel comprend :
la détection du niveau d'activité du patient par le capteur de mouvement (101) intégré au système CGM (1) ;
la transmission au processeur (102) intégré au système CGM (1) des signaux indiquant le niveau d'activité du patient dans des positions spécifiques, fournis par le capteur de mouvement (101) intégré au système CGM (1), lesdites positions spécifiques comprenant la position debout, assise, couchée ou le passage d'une de ces positions à une autre ;
déterminer l'état physique du patient en fonction du niveau d'activité via le processeur (202) intégré à la pompe patch (2) ;
ajuster une pluralité d'algorithmes via le processeur (102) intégré au système CGM (1), en fonction, entre autres, des signaux provenant du capteur de mouvement (101) intégré au système CGM (1) ;
envoyer, à partir du processeur (102) intégré au système CGM (1), des instructions correspondantes basées sur les algorithmes ajustés vers le processeur (202) intégré à la pompe-patch (2) pour assurer le fonctionnement automatique du pancréas artificiel ; et la commutation automatique du pancréas artificiel entre différents modes de fonctionnement, sur la base, au moins en partie, des algorithmes ajustés,
dans lequel la pluralité d'algorithmes comprend un algorithme de suspension en cas d'hypoglycémie, un algorithme de suspension prédictive en cas d'hypoglycémie et un algorithme de seuil d'alerte.

2. Pancréas artificiel en boucle fermée selon la revendication 1, dans lequel,
la pompe patch (2) et le système CGM (1) sont séparés l'un de l'autre.

3. Pancréas artificiel en boucle fermée selon la revendication 1, dans lequel :
la pomp patch (2) et le système CGM (1) sont intégrés dans un pancréas artificiel à aiguille unique ;
le capteur de mouvement (101) et les processeurs (102, 202) sont intégrés dans le pancréas artificiel à aiguille unique.

4. Pancréas artificiel en boucle fermée selon la revendication 1, dans lequel,
le capteur de mouvement (101) comprend un ou plusieurs éléments parmi un accéléromètre, un gyroscope et un capteur d'orientation.

5. Pancréas artificiel en boucle fermée selon la revendication 1, dans lequel les algorithmes sont ajustés en fonction des différentes intensités d'effort lorsque le patient est en train de faire de l'exercice physique.

6. Pancréas artificiel en boucle fermée selon la revendication 1, dans lequel le pancréas artificiel en boucle fermée passe automatiquement en mode « mode silencieux» pour les alertes de faible priorité lorsqu'il est déterminé que le patient est en état de sommeil ou d'exercice physique.
